Europäisches Patentamt

European Patent Office

Office européen des brevets

(19)

(11) Veröffentlichungsnummer : **0 142 742**
**B1**

(12) **EUROPÄISCHE PATENTSCHRIFT**

(45) Veröffentlichungstag der Patentschrift :
30.11.88

(51) Int. Cl.⁴ : **C 07 D265/30**

(21) Anmeldenummer : **84112961.2**

(22) Anmeldetag : **27.10.84**

(54) Verfahren zur Entwässerung von 2,6-Dimethylmorpholin.

(30) Priorität : 22.11.83 DE 3342009

(43) Veröffentlichungstag der Anmeldung :
29.05.85 Patentblatt 85/22

(45) Bekanntmachung des Hinweises auf die Patenterteilung : 30.11.88 Patentblatt 88/48

(84) Benannte Vertragsstaaten :
BE CH DE FR GB IT LI NL

(56) Entgegenhaltungen :
EP-A- 0 026 367
US-A- 3 083 202
Azeotropic Data III, Adv. Chem. Ser. 116, 1973, S.626-628
Die Akte enthält technische Angaben, die nach dem Eingang der Anmeldung eingereicht wurden und die nicht in dieser Patentschrift enthalten sind.

(73) Patentinhaber : HÜLS AKTIENGESELLSCHAFT
Patentabteilung / PB 15 - Postfach 13 20
D-4370 Marl 1 (DE)

(72) Erfinder : Müller, Wolfgang Hans Eduard, Dr.
Kaspar-Grove Strasse 32
D-4370 Marl (DE)
Erfinder : Böxkes, Werner, Dr.
Oppauer Strasse 2
D-4370 Marl (DE)
Erfinder : Scholten, Heinz, Dr.
Rehwinkel 3
D-4358 Haltern 7 (DE)

EP 0 142 742 B1

**Beschreibung**

Die Erfindung betrifft die Isolierung bzw. Wiedergewinnung von praktisch trockenem 2,6-Dimethylmorpholin aus wasserhaltigen Lösungen.

Im Stand der Technik wird für die Trennung von Gemischen aus Wasser/2,6-Dimethylmorpholin die Azeotroprektifikation mit einem Schleppmittel, wie z. B. Benzol (US-PS 3 083 202), angegeben. Daneben wird für die Entwässerung von Alkylmorpholin auch die Flüssig-flüssig-Extraktion, mit z. B. Ether, genannt (J. Org. Chem. 11 (1946), 286 bis 291 ; C.A. 40, 4732, 1 bis 7). Auch die in DE-OS 19 18 388 beschriebene Aufarbeitung zu 2,6-Dimethylmorpholin (Beispiele 4 bis 6) erfolgt in Gegenwart von Hilfsstoffen. Als Hilfsstoffe fungieren die von der Synthese des 2,6-Dimethylmorpholin im Rohprodukt in erheblicher Konzentration anwesenden Stoffe Diisopropanolamin und Diisopropanolaminhydrochlorid.

Nach dem Stand der Technik können Wasser und 2,6-Dimethylmorpholin durch einfache Rektifikation, d. h. ohne Anwendung von Hilfsstoffen, nicht getrennt werden, da sie vollständig mischbar sind und ein Azeotrop (70 Gew.-% Wasser, Kp 99,6 °C) bilden (Azeotropic data III, System Nr. 567).

Die Schleppmittelrektifikation zur Trocknung von mit Wasser vollständig mischbaren Flüssigkeiten ist technisch bewährt. Sie hat jedoch folgende Nachteile : Es wird eine zusätzliche Kolonne als Abwasserstripper benötigt, da aus ökologischen Gründen kein schleppmittelgesättigtes Wasser an die Umwelt abgegeben werden darf. Die Einführung eines Hilfsstoffes erfordert eine zusätzliche Vorratshaltung mit allen dabei entstehenden Nachteilen. Ferner besteht die Möglichkeit der Verunreinigung des Produktes durch den Hilfsstoff sowie die der Anreicherung von in kleinen Mengen vorhandenen Nebenprodukten im Schleppmittelkreislauf, was zu Komplikationen führen kann. Weiterhin besteht die Notwendigkeit, große Mengen Schleppmittel zu verdampfen und zu kondensieren. Die Verdampfung des Schleppmittels erfordert einen zusätzlichen beträchtlichen Energiebedarf.

Es stellte sich die Aufgabe, nach einem einfachen, wirtschaftlichen Verfahren zur Trennung von Wasser und 2,6-Dimethylmorpholin zu suchen, das die genannten Nachteile nicht aufweist.

Da allein praktisch wasserfreies 2,6-Dimethylmorpholin handelsüblich ist bzw. für weitere Umsetzungen oder für die bekannte Trennung in die cis-/trans-Isomeren eingesetzt wird, war Voraussetzung, daß nach der Trennung der beiden Komponenten Wasser/2,6-Dimethylmorpholin ein 2,6-Dimethylmorpholin mit einem Wassergehalt von maximal 500 Gew.-ppm vorliegt. Die Auftrennung der Komponenten sollte aus Gemischen mit beliebigem Wassergehalt durchführbar sein.

Die Lösung der gestellten Aufgabe erfolgte anspruchsgemäß.

Der aufgefundene Lösungsweg war für den Fachmann überraschend, da bekannterweise Stoffe umso leichter miteinander Azeotrope bilden, je geringer die Differenz ihrer Siedepunkte ist (z. B. L.H. Horsley in « Azeotropic Data III », Seite 615, Washington D.C., 1973). Für das System Wasser/2,6-Dimethylmorpholin ergibt sich aus den literaturbekannten Dampfdruckangaben (DE-OS 29 38 698 und H. Booth, G.C. Gidley : Tetrahedron, 21 (1965), Seiten 3429 bis 3434) ein größerer Abstand der Siedepunkte bei höheren Drücken als bei niedrigen. Deshalb war — wenn überhaupt — ein Verschwinden der Azeotropie eher bei höheren Drücken als bei niedrigeren Drücken zu erwarten. Die gefundene Lösung der Aufgabe war somit keineswegs vorhersehbar.

Das erfindungsgemäße Verfahren kann diskontinuierlich oder kontinuierlich durchgeführt werden. Die Rektifikation erfolgt bei Drücken am Kolonnenkopf von 6 bis 150 hPa (mbar), vorteilhaft bei Drücken von 30 bis 100 hPa (mbar). Rektifikationskolonnen üblicher Bauart sind geeignet, besonders vorteilhaft sind Kolonnen mit geringen Druckverlusten je Trennstufe (≤ 3,5 hPa (mbar) je Trennstufe) im Verstärkungsteil der Kolonne, während der Druckverlust des Abtriebteiles der Kolonne von geringerer Bedeutung ist. Es können sowohl Boden- als auch Füllkörperkolonnen sowie Kolonnen mit anderen Einbauten angewendet werden.

Das Verfahren kann bei allen Gemischen aus Wasser und 2,6-Dimethylmorpholin angewendet werden, z. B. bei solchen, die bei der Synthese von 2,6-Dimethylmorpholin aus Bis-2-oxypropylamin mit Schwefelsäure nach der Abtrennung der Schwefelsäure als Natriumsulfat anfallen. Geringere Anteile von mit Wasser azeotrop siedende Gemische bildende sonstige Stoffe stören nicht ; sie können zusammen mit dem Wasser abgetrennt werden, wodurch reineres 2,6-Dimethylmorpholin als bei der Entwässerung nach anderen Methoden erhalten werden kann.

Das Verfahren der Erfindung besitzt gegenüber den aus dem Stand der Technik bekannten Verfahren, bei denen mit Hilfsstoffen gearbeitet wird, eine Reihe von Vorteilen. Die Investitionskosten sind wegen der Einsparung einer Kolonne niedriger, der Energieverbrauch ist geringer, die Reinheit des erhaltenen Produktes ist größer und Nebenbestandteile im Rohprodukt sind weniger störend. Der Wassergehalt im erfindungsgemäß rektifizierten 2,6-Dimethylmorpholin beträgt maximal 500 Gew.-ppm ; bei entsprechender Auslegung der Rektifikation können Gew.-ppm-Werte < 1 erreicht werden.

Die nachstehenden Beispiele dienen der Erläuterung des Verfahrens.

Die in der Tabelle 1 angegebene Versuchsreihe soll den Einfluß des Kopfdruckes bei der Rektifikation aufzeigen. Es wurde versucht, alle Parameter, mit Ausnahme des Kopfdruckes, weitgehend konstant zu lassen.

Durch diese Darstellung wird der bestehende Zusammenhang von Kopfdruck und Rücklaufverhältnis deutlich : Je geringer der Kopfdruck, desto geringer das erforderliche Rücklaufverhältnis. Bei einem Kopfdruck von 200 hPa (mbar) ist das Rücklaufverhältnis bereits so groß, daß das Verfahren unwirtschaftlich wird. Bei einem Druck von 300 hPa (mbar) kann die gewünschte Trennung nicht mehr erreicht werden. Bei einem Kopfdruck < 6 hPa (mbar) ist die Trennaufgabe nicht mehr lösbar, weil der Rücklauf gefrieren würde. Die Siedetemperatur beträgt bei 6 hPa (mbar) ca. 0 °C.

Dem Fachmann ist es geläufig, daß für die Darstellung des Zusammenhanges z. B. auch ein bestimmtes Rücklaufverhältnis von 3 hätte konstant gehalten werden können, bei der Variation der Bodenzahl der Rektifikationskolonne. Auch dabei wäre — wie in unseren Versuchen — praktisch immer die gleiche Konzentration am Kolonnenkopf und am Kolonnensumpf erhalten worden. Allerdings ist diese Methode mit einem größeren experimentellen Aufwand verbunden, ohne daß dadurch weitergehende Erkenntnisse erzielbar sind. Eine weitere Möglichkeit ist, Bodenzahl und Rücklaufverhältnis konstant zu halten und die Abhängigkeit der erhaltenen Konzentration vom Kopfdruck zu bestimmen. Auch diese Methode bietet keine besseren Einblicke.

Tabelle 1 Beispiele für kontinuierliche Rektifikation von Gemischen aus Wasser und cis-2,6-Dimethylmorpholin
(Kolonne mit 40 praktischen Böden, davon 24 im Verstärkungsteil)

| Beispiel Nr. | Druck am Kolonnenkopf hPa (mbar) | Differenz-druck hPa (mbar) | erforderliches Rücklauf-verhältnis | Konzentration (Gew. — % Wasser im | | |
| --- | --- | --- | --- | --- | --- | --- |
| | | | | Zulauf | Destillat | Sumpfprodukt |
| 1 | 10 | 140 | 0,8 | 75,0 | 99,96 | 0,010 |
| 2 | 30 | 140 | 1,0 | 75,0 | 99,80 | 0,005 |
| 3 | 50 | 140 | 1,1 | 75,0 | 99,88 | 0,008 |
| 4 | 75 | 140 | 1,4 | 75,0 | 99,91 | 0,012 |
| 5 | 100 | 140 | 1,7 | 75,0 | 99,88 | 0,003 |
| 6 | 150 | 140 | 2,6 | 75,0 | 99,75 | 0,009 |
| 7 | 200 | 140 | 4,1 | 75,0 | 99,89 | 0,005 |
| 8 | 300 | 140 | | 75,0 | *) | 0,016 |

*) Destillatkonzentration von ~ 99,9 Gew.-% Wasser konnte nicht erreicht werden

Mit den von uns angegebenen Versuchsparametern ist der Gegenstand der Erfindung hinreichend offenbart. Bei Kenntnis des Dampf-/Flüssigkeitsgleichgewichtes für den gewählten Druck innerhalb des erfindungsgemäßen Bereiches ist es dem mit einschlägigen Aufgaben betrauten Fachmann möglich, die Rektifikationskolonne für die Trennung eines beliebigen Gemisches 2,6-Dimethylmorpholin/Wasser in gängiger Weise auszulegen.

In der Tabelle 2 sind die Ergebnisse von drei kontinuierlichen Rektifikationen von technischen Reaktionsprodukten, die bei der Dimethylmorpholin-Synthese erhalten wurden, angegeben, wobei neben cis- und trans-2,6-Dimethylmorpholin und cis- und trans-2,5-Dimethylmorpholin auch eine Reihe weiterer, z. T. unbekannter, Stoffe in geringerer Menge anwesend waren, die sich auf Destillat und Sumpfprodukt verteilten. Wie die Analysen zeigen, werden diese Stoffe beim erfindungsgemäßen Arbeiten bevorzugt zusammen mit dem Wasser vom zu gewinnenden 2,6-Dimethylmorpholin abgetrennt, was zu einer verbesserten Produktreinheit führt.

Vergleichsbeispiel 11 zeigt, daß im Falle eines Kopfdruckes von > 150 hPa (mbar) im Kopfprodukt (Destillat) relativ hohe Dimethylmorpholin-Gehalte vorhanden sind, d. h., die Trennung ist nicht mehr befriedigend.

Tabelle 2 Beispiele 9 und 10 sowie Vergleichsbeispiel 11 für kontinuierliche Rektifikation von technischen Gemischen

| Beispiel Nr. | 9 | 10 | 11 |
| --- | --- | --- | --- |
| Gesamtzahl der Böden | 40 | 40 | 40 |
| Böden im Verstärkungsteil | 30 | 30 | 30 |
| Rücklaufverhältnis | 1,33 | 1,0 | 1,33 |
| Druck am Kolonnenkopf (hPa) (mbar) | 74 | 75 | 160 |
| Druck am Kolonnensumpf (hPa) (mbar) | 130 | 125 | 212 |
| Temp. am Kolonnenkopf (°C) | 41 | 40 | 56 |
| Temp. am Kolonnensumpf (°C) | 85 | 84 | 98 |

**0 142 742**

Tabelle 2  (Fortsetzung)

| Beispiel Nr. | 9 | 10 | 11 |
|---|---|---|---|
| Menge Destillat, bezogen auf Zulauf (%) | 59,3 | 59,7 | 60,3 |
| Analyse Zulauf (Gew. – %) | | | |
| Wasser | 59 | 59,5 | 59 |
| 2,5- und 2,6-Dimethylmorpholine | 40,0 | 39,55 | 40,0 |
| sonstige Stoffe | 1,0 | 0,95 | 1,0 |
| Analyse Destillat (Gew. – %) | | | |
| Wasser | 99,3 | 99,1 | 97,7 |
| 2,5- und 2,6-Dimethylmorpholine | 0,077 | 0,15 | 1,62 |
| sonstige Stoffe | 0,61 | 0,72 | 0,70 |
| Analyse Sumpfprodukt (Gew. – %) | | | |
| Wasser | 0,03 | 0,04 | 0,03 |
| 2,5- und 2,6-Dimethylmorpholine | 98,44 | 98,60 | 98,95 |
| sonstige Stoffe | 1,49 | 1,31 | 1,02 |

**Patentansprüche**

1. Verfahren zur diskontinuierlichen bzw. kontinuierlichen Entwässerung von 2,6-Dimethylmorpholin/Wasser-Gemischen mit beliebigem Wassergehalt durch Zerlegung unter Wärmezufuhr, dadurch gekennzeichnet, daß die Abtrennung des Wassers vom 2,6-Dimethylmorpholin durch Rektifikation in einer Kolonne üblicher Bauart bei Drücken von 6 bis 150 hPa (mbar) am Kolonnenkopf erfolgt, wobei das entwässerte 2,6-Dimethylmorpholin im Sumpf anfällt.

2. Verfahren nach Anspruch 1, dadurch gekennzeichnet, daß der Druck am Kolonnenkopf 30 bis 100 hPa (mbar) beträgt.

3. Verfahren nach Anspruch 1 und 2, dadurch gekennzeichnet, daß das Verfahren im Verstarkungsteil der Kolonne mit einem Druckverlust von ≤ 3,5 hPa (mbar) je Trennstufe durchgeführt wird.

**Claims**

1. A process for the discontinuous or continuous dehydration of 2,6-dimethylmorpholine/water mixtures of any water content by separation with supply of heat, characterized in that the separation of the water from the 2,6-dimethylmorpholine is effected by rectification in a column of conventional construction under pressures from 6 to 150 hPa (mbar) at the column top, the dehydrated 2,6-dimethylmorpholine being obtained at the bottom.

2. A process according to claim 1, characterized in that the pressure at the column top is 30 to 100 hPa (mbar).

3. A process according to claims 1 and 2, characterized in that the process is carried out at a pressure drop of ≤ 3.5 hPa (mbar) per separation stage in the rectifying section of the column.

**Revendications**

1. Procédé pour la déshydratation discontinue ou continue de mélanges de 2,6-diméthyl-morpholine et d'eau présentant une teneur quelconque en eau, par décomposition avec rapport de chaleur, caractérisé par le fait que la séparation de l'eau de la 2,6-diméthyl-morpholine a lieu par rectification dans une colonne d'un mode de construction usuel sous des pressions de 6 à 150 hPa (m.bars) à la tête de la colonne, la 2,6-diméthyl-morpholine déshydratée se rassemblant dans le pied de la colonne.

2. Procédé selon la revendication 1, caractérisé par le fait que la pression à la tête de la colonne est de 30 à 100 hPa (m.bars).

3. Procédé selon la revendication 1 ou 2, caractérisé par le fait que le procédé est mis en œuvre dans la partie de renforcement de la colonne avec une perte de pression inférieure ou égale à 3,5 hPa (m.bars) par étage de séparation.

4